(19) Europäisches Patentamt
European Patent Office
Office européen des brevets

(11) Veröffentlichungsnummer : **0 479 727 A1**

# (12) EUROPÄISCHE PATENTANMELDUNG

(21) Anmeldenummer : **91810755.8**

(22) Anmeldetag : **26.09.91**

(51) Int. Cl.[5] : **C09B 57/04,** C07D 209/50, C09B 56/00, C09B 23/10

(30) Priorität : **05.10.90 CH 3218/90**

(43) Veröffentlichungstag der Anmeldung : **08.04.92 Patentblatt 92/15**

(84) Benannte Vertragsstaaten : **CH DE FR GB LI**

(71) Anmelder : **CIBA-GEIGY AG**
**Klybeckstrasse 141**
**CH-4002 Basel (CH)**

(72) Erfinder : **Ruf, Klaus, Dr.**
**Eckstrasse 24**
**W-7801 Bollschweil (DE)**

## (54) Verfahren zur Herstellung von Bisisoindolinonpigmenten.

(57) Verfahren zur Herstellung von Isoindolinonpigmenten der Formel

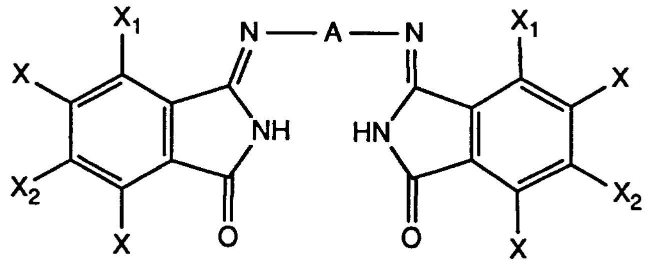

durch Umsetzung von 2 Mol eines Isoindolinons der Formel

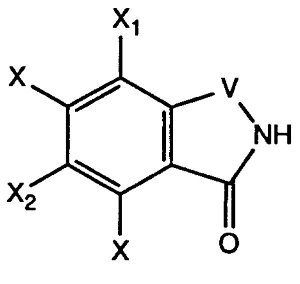

mit einem Mol eines Diamins der Formel

$$H_2N\text{-}A\text{-}NH_2,$$

dadurch gekennzeichnet, dass die Umsetzung in Gegenwart von 1,2,3-Trimethylbenzol als Lösungsmittel durchgefütirt wird.

Für die Bedeutung der Substituenten A, X, $X_1$, $X_2$ und V wird auf Anspruch 1 verwiesen.

EP 0 479 727 A1

Die vorliegende Erfindung betrifft ein Verfahren zur Herstellung von Bisisoindolinonpigmenten nach an sich bekannten Methoden, aber unter Verwendung von 1,2,3-Trimethylbenzol als Lösungsmittel.

Bisisoindolinonpigmente sind schon lange bekannt und Verfahren zu ihrer Herstellung durch Kondensation bestimmter Isoindolinone, wie z.B. solchen der Formel

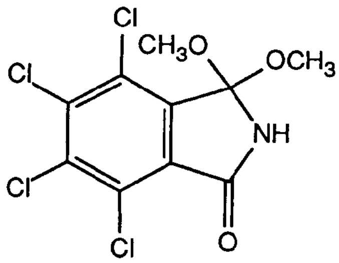 , 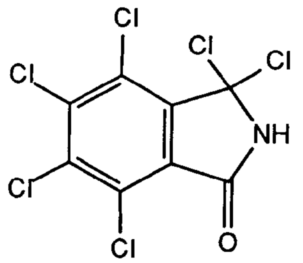 oder 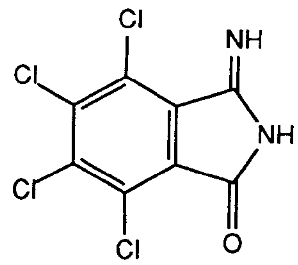 ,

mit Diaminen, sind in einer grossen Zahl von Publikationen beschrieben, so z.B. in den US-Patenten 2 973 358, 3 971 805, 4 006 162, 4 043 999, 4 231 931 und 4 327 024. Die Kondensation wird dabei bevorzugt in o-Dichlorbenzol als Lösungsmittel durchgeführt, wie dies im allgemeinen auch im grosstechnischen Massstab geschieht. In den obenerwähnten Publikationen wird allerdings erwähnt und an einigen Beispielen gezeigt, dass auch andere Lösungsmittel geeignet sind, darunter z.B. Xylol (US 2 973 358, Beispiel 4).

Es ist nun gefunden worden, dass bei Verwendung von 1,2,3-Trimethylbenzol als Lösungsmittel für die obenerwähnte Kondensationsreaktion Produkte erhalten werden, die sich überraschenderweise durch bessere Eigenschaften und insbesondere durch höhere Reinheit gegenüber denjenigen auszeichnen, die beim Einsatz anderer unchlorierter Lösungsmittel und auch von o-Dichlorbenzol erhalten werden.

Die vorliegende Erfindung betrifft demnach ein Verfahren zur Herstellung von Isoindolinonpigmenten der Formel

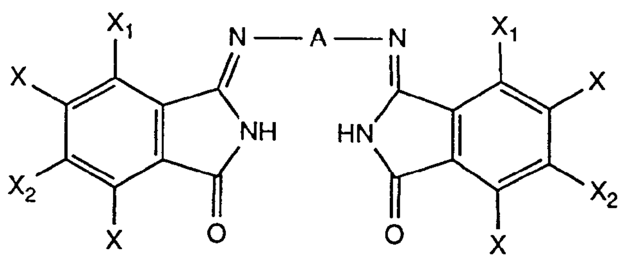 (I),

worin A eine der Gruppen der Formeln

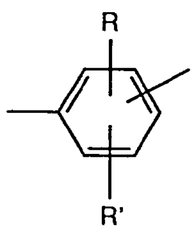 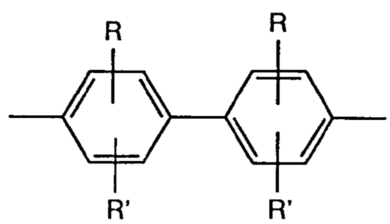 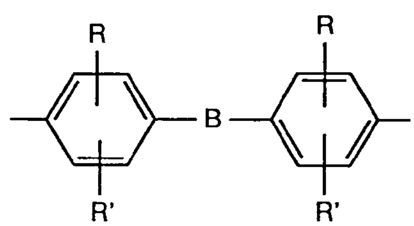

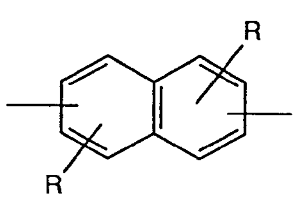 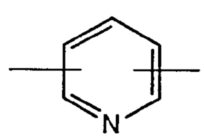 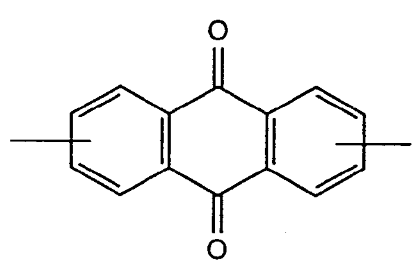

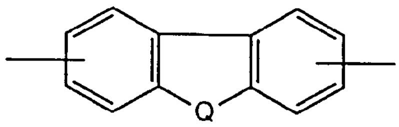 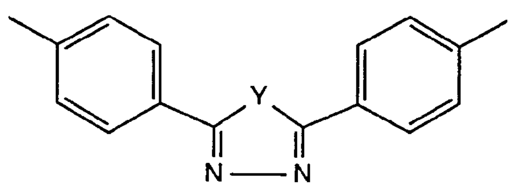

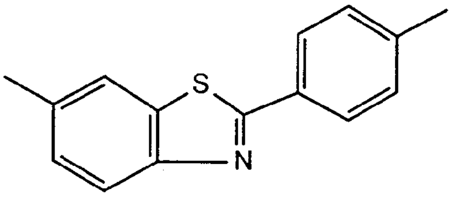 oder 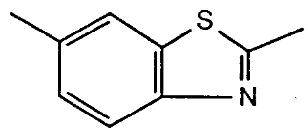

ist, worin B -O-, -S-, $-SO_2-$, N=N-, $-CH_2-$, -CH=CH-,

$$-O-(CH_2)_n-O-,$$

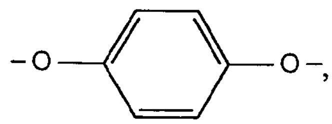

-CONH-, -NHCONH-, -NHCOCONH- oder -CONHNHCO-und n eine Zahl von 1 bis 4 bedeuten, Q -O- oder -S- ist und die R und R′ unabhängig voneinander Wasserstoff, Halogen, $C_1$-$C_4$-Alkyl, $C_1$-$C_4$-Alkoxy oder Phenoxy, X Halogen und $X_1$ und $X_2$ Halogen oder $C_1$-$C_4$-Alkoxy bedeuten,
durch Umsetzung von 2 Mol eines Isoindolinons der Formel

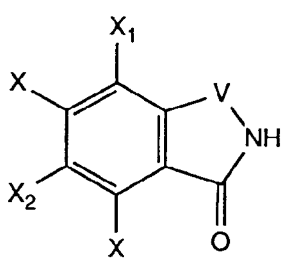 (II),

worin X, $X_1$ und $X_2$ die oben angegebene Bedeutung haben und V eine Gruppe der Formel

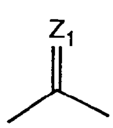 oder 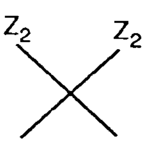

bedeutet, worin $Z_1$ für eine Imino- oder Thiogruppe und $Z_2$ für Chlor, Brom, $C_1$-$C_4$-Alkoxy oder eine sekundäre Aminogruppe stehen,
mit einem Mol eines Diamins der Formel

$$H_2N\text{-}A\text{-}NH_2 \qquad \text{(III)},$$

worin A die oben angegebene Bedeutung hat,
dadurch gekennzeichnet, dass die Umsetzung in Gegenwart von 1,2,3-Trimethylbenzol als Lösungsmittel durchgeführt wird.

Bedeuten etwaige Substituenten Halogen, so handelt es sich dabei z.B. um Fluor, vorzugsweise Brom und insbesondere Chlor.

Bedeutet R oder R′ $C_1$-$C_4$-Alkyl, so meint man damit z.B. Methyl, Ethyl, n-Propyl, Isopropyl, n-Butyl, sec.-Butyl oder tert.-Butyl. Bevorzugt ist Methyl.

R, R′, $X_1$, $X_2$ und $Z_2$ stellen als $C_1$-$C_4$-Alkoxy z.B. Methoxy, Ethoxy, n-Propoxy, n-Butoxy oder sec-Butoxy

dar. Bevorzugt ist Methoxy.

Als eine Gruppe der Formel

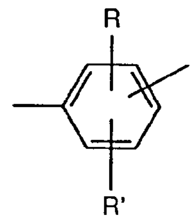

stellt A beispielsweise die unsubstituierte p-oder m-Phenylengruppe oder die 2-Chlor-1,4-phenylen-, 2-Methyl-1,4-phenylen-, 2-Ethyl-1,4-phenylen-, 2-n-Propyl-1,4-phenylen-, 2-Isopropyl-1,4-phenylen-, 2-tert.-Butyl- 1,4-phenylen-, 2-Methoxy- 1,4-phenylen-, 2-Ethoxy- 1,4-phenylen-, 2-tert.-Butoxy-1,4-phenylen-, 2,5-Dichlor- 1,4-phenylen-, 2,5-Dimethyl- 1,4-phenylen-, 2,5-Diethyl- 1,4-phenylen-, 2,5-Dimethoxy- 1,4-phenylen-, 2,5-Diethoxy- 1,4-phenylen-, 2-Chlor-5-methyl-1,4-phenylen-, 2-Chlor-5-methoxy- 1,4-phenylen-, 2-Methyl-5-methoxy- 1,4-phenylen-, 2,6-Dichlor- 1,4-phenylen-, 2,6-Dimethyl- 1,4-phenylen-, 2-Chlor-6-methoxy- 1,4-phenylen-, 2-Chlor- 1,3-phenylen-, 2-Methyl- 1,3-phenylen-, 2-Methoxy- 1,3-phenylen-, 2,5-Dichlor- 1,3-phenylen-, 2,5-Dimethyl- 1,3-phenylen- oder 2-Chlor-5-methyl- 1,3-phenylengruppe, insbesondere jedoch eine mit Chloratomen, Methyl-, Methoxy- oder Ethoxygruppen in 2-Stellung monosubstituierte oder in 2,5-Stellung gleich oder verschieden mit Chloratomen, Methyl-, Methoxy- oder Ethoxygruppen disubstituierte 1,3-oder 1,4-Phenylengruppe dar. Bevorzugt ist die 2-Methyl- 1,3-phenylengruppe.

Bedeutet A eine Gruppe der Formel

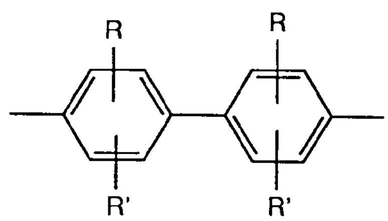,

so handelt es sich beispielsweise um die unsubstituierte 4,4′-Biphenylylengruppe, oder die 3,3′-Dichlor-, 3,3′-Dimethyl-, 3,3′-Diethyl-, 3,3′-Diisopropyl-, 3,3′-Di-n-butyl-, 3,3′-Di-tert.-butyl-, 3,3′-Dimethoxy-, 3,3′-Diethoxy-, 3,3′-Di-tert.-butoxy-, 3,3′,5,5′-Tetrachlor-, 3,3′,5,5′-Tetramethyl-, 3,3′,5,5′-Tetramethoxy-, 3,3′-Dichlor-5,5′-dimethyl-, 3,3′-Dichlor-5,5′-dimethoxy-, 3,3′-Dimethyl-5,5′-methoxy-4,4′-biphenylylengruppe, insbesondere jedoch um eine mit Chloratomen, Methyl-, Methoxy- oder Ethoxygruppen in 3,3′-Stellung gleich disubstituierte 4,4′-Biphenylylengruppe.

Stellt A eine Gruppe der Formel

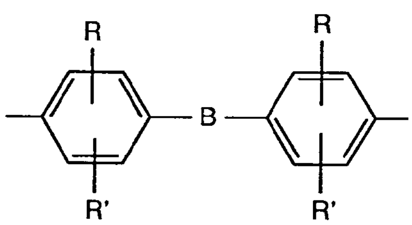 dar,

so handelt es sich beispielsweise um gegebenenfalls entsprechend den obengenannten Biphenylylenderivaten substituierte, vorzugsweise jedoch um unsubstituierte 4,4′- Diphenyl-ylenether-, 4,4′-Diphenylylensulfid-, 4,4′-Diphenylylen-sulfon-, 4,4′-Diphenylylen- azo-, 2-Chlor-4,4′-diphenylylen-azo-, 3-Chlor-4,4′-diphenylylen-azo-, 2-Methyl-4,4′-diphenylylen-azo-, 3-Methyl-4,4′-diphenylylen-azo-, 2-Methoxy-4,4′-diphenylylen-azo-, 3-Methoxy-4,4′-diphenylylen-azo-, 4,4′-Diphenylylen-methan-, 4,4′-Diphenylylen-ethen-, 4,4′-Diphenylylen-dioxymethan-, 4,4′-Diphenylylen-dioxy-benzol-, 4,4′-Diphenylylen-carbamid-, 4,4′-Diphenylylen-harnstoff oder 4,4′-Diphenylylen-dicarbamyl-hydrazingruppen.

Bevorzugt ist die 2-Methyl-4,4′-diphenylylen-azogruppe.

Weiter bedeutet A z.B. gegebenenfalls entsprechend den obengenannten Biphenylylen-derivaten substituiertes, vorzugsweise jedoch unsubstituiertes 1,4-, 1,5- oder 2,6-Naphthylen, 1,5-Anthrachinondiyl, 1,4-Anthrachinondiyl, 2,6-Pyridindiyl, 2,7-Carbazoldiyl, 2,8-Dibenzofurandiyl, 3,8-Dibenzofurandiyl, 3,7-Dibenzofurandiyl, 6,4′-(2-Phenyl)-benzthiazoldiyl, 2,6-Benzthiazoldiyl, 4′,4″-(2,5-Bisphenyl)-oxadiazoldiyl oder 4′,4″-(2,5-Bisphenyl)-thiadiazoldiyl.

A stellt vorzugsweise eine Gruppe der Formel

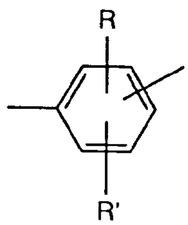
oder
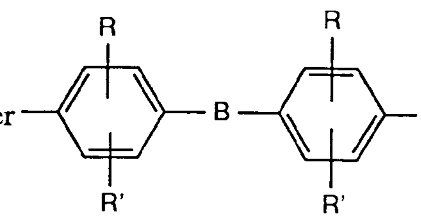

dar, worin die R und R′ die oben angegebene Bedeutung haben und B -N=N-, -CONH-, - NHCONH- oder - CONHNHCO- bedeutet und insbesondere eine Gruppe der Formel

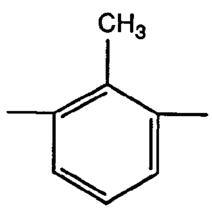
oder
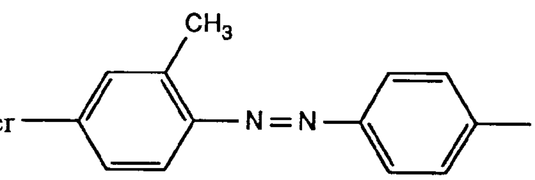

Bevorzugte Isoindolinone der Formel II sind diejenigen, worin X, $X_1$ und $X_2$ Chlor bedeuten sowie jene, worin V eine Gruppe der Formel

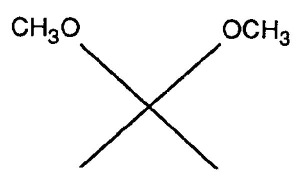

ist.

Die Isoindolinone der Formel II und die Diamine der Formel III sind bekannte Verbindungen.

Abgesehen von der Verwendung von 1,2,3-Trimethylbenzol als Lösungsmittel, erfolgt die Kondensation nach bekannten und allgemein üblichen Methoden. Zweckmässig wird bei Temperaturen zwischen 40 und 100°C, bevorzugt zwischen 70 und 90°C, bei einer Dauer von 2 bis 5, vorzugsweise $2\frac{1}{2}$ bis 4 Stunden, gearbeitet. Um die gewünschte Reinheit, Kristallinität und Partikelgrösse der Pigmente zu erhalten, wird anschliessend z.B. auf über 130°C, bevorzugt zwischen 140 und 160°C, erhitzt. Die erhaltenen Produkte sind für gewisse Zwecke als Rohpigmente direkt verwenbar; sie können aber auch nach an sich bekannten Methoden konditioniert werden.

Zweckmässig wird die 3- bis 10-fache, vorzugsweise die 5- bis 8-fache Gewichtsmenge 1,2,3-Trimethylbenzol, bezogen auf das Isoindolinon der Formel II, eingesetzt.

Die nächstfolgenden Beispiele erläutern die Erfindung.

<u>Beispiel 1:</u> In einem 1,5-Liter Planschliffkolben werden 500 ml 1,2,3-Trimethylbenzol vorgelegt und 116,2 g (0,374 Mol) 3,4,5,6-Tetrachlor-2-cyan-benzoesäuremethylester (96,2 %ig) eingetragen. Innerhalb von 10 Min. lässt man 70,7 g Natriummethylat (30 % in Methanol) zulaufen. Anschliessend erhitzt man auf 60-65°C und lässt eine Stunde rühren, wobei sich das gewünschte Isoindolinon bildet.

Gleichzeitig werden in einem 500 ml Erlenmeyer-Kolben 20,8 g (0,167 Mol) 2,6-Diaminotoluol (98,1 %ig) in 400 ml 1,2,3-Trimethylbenzol bei 85°C gelöst. Nach Eintragen von 1 g Aktivkohle wird klärfiltriert. Die helle 2,6-Diaminotoluollösung wird anschliessend der Isoindolinonlösung zugegeben. Man erwärmt auf 80°C und rührt 3 Stunden.

Dann wird vorsichtig mittels Wasserstrahlvakuum evakuiert und Methanol bei 73-77°C abdestilliert. In die orangene dickflüssige Suspension werden 68 ml Eisessig eingetropft. Die gelbe Pigmentsuspension wird innerhalb von 60 Minuten auf 150-155°C erhitzt und zwei Stunden bei dieser Temperatur verrührt. Man lässt auf 100°C abkühlen und lässt 500 ml Methanol zufliessen. Man filtriert und wäscht das Nutschgut mit 800 ml Methanol und 1000 ml Wasser. Das Pigment wird bei 100°C bis zur Gewichtskonstanz getrocknet.
Ausbeute: 108 g (98,6 % d. Th.).

Beispiel 2: Man legt 1026 ml 1,2,3-Trimethylbenzol in einem 3-Liter Planschliffkolben vor, gibt 166,5 g (0,557 Mol) 3,4,5,6-Tetrachlor-2-cyan-benzoesäuremethylester (96,2 %ig) dazu und lässt bei Raumtemperatur rühren. 58 g 4,4'Diamino-2-methyl-azo-benzol (0,257 Mol) werden gleichzeitig in 250 ml Methanol verrührt und filtriert. Man gibt das Nutschgut zusammen mit weiteren 440 ml Methanol dazu.

Anschliessend werden 100 g Natriummethylat (30 % in Methanol) eingetragen. Dann erwärmt man auf 60-65°C, destilliert drei Stunden Methanol ab und ersetzt diesen gleichzeitig mit 400 ml 1,2,3-Trimethylbenzol. Während dieser Destillation erhöht man die Temperatur kontinuierlich auf 85°C. Dann werden 65 g Eisessig zugetropft. Man erhitzt auf 150°C und lässt zwei Stunden bei dieser Temperatur verrühren. Nach dem Abkühlen auf 100°C werden 700 ml Methanol dazugesetzt. Man filtriert und wäscht mit 1200 ml Methanol und 1500 ml Wasser. Das Pigment wird bei 120°C bis zur Gewichtskonstanz getrocknet.
Ausbeute: 181 g (92,6 % d. Th.).

## Patentansprüche

1. Verfahren zur Herstellung von Isoindolinonpigmenten der Formel I

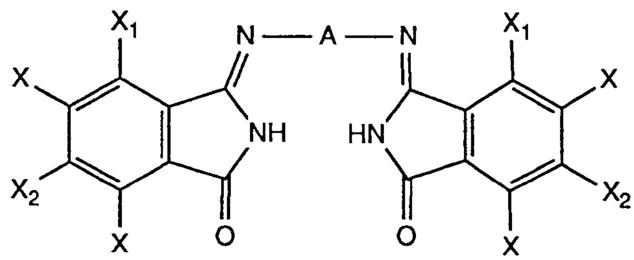

(I),

worin A eine der Gruppen der Formeln

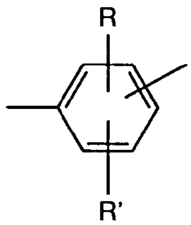
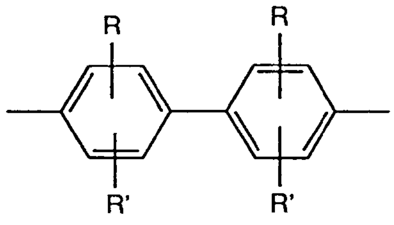
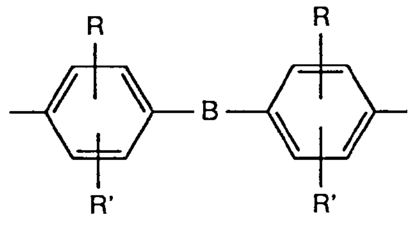

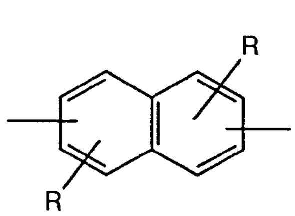
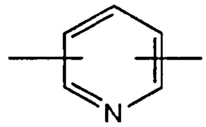
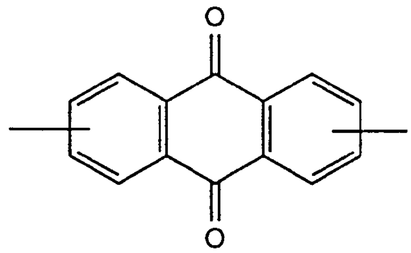

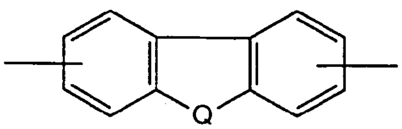
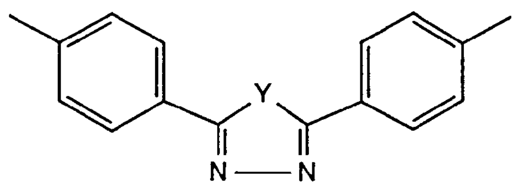

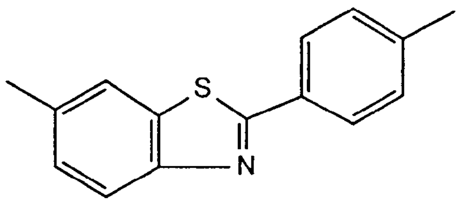
oder
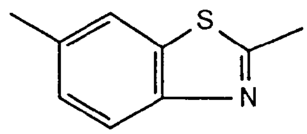

ist, worin B -O-, -S-, $-SO_2-$, N=N-, $-CH_2-$, -CH=CH-,

$$-O-(CH_2)_n-O-,$$

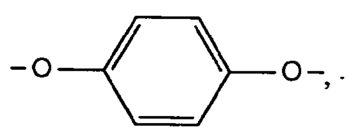

-CONH-, -NHCONH-, -NHCOCONH- oder -CONHNHCO- und n eine Zahl von 1 bis 4 bedeuten, Q -O- oder -S- ist und die R und R′ unabhängig voneinander Wasserstoff, Halogen, $C_1$-$C_4$-Alkyl, $C_1$-$C_4$-Alkoxy oder Phenoxy, X Halogen und $X_1$ und $X_2$ Halogen oder $C_1$-$C_4$-Alkoxy bedeuten,
durch Umsetzung von 2 Mol eines Isoindolinons der Formel

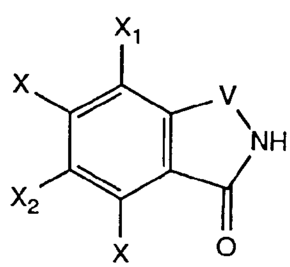
(II),

worin X, $X_1$ und $X_2$ die oben angegebene Bedeutung haben und V eine Gruppe der Formel

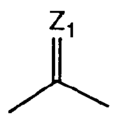 oder 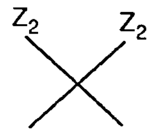

bedeutet, worin $Z_1$ für eine Imino- oder Thiogruppe und $Z_2$ für Chlor, Brom, $C_1$-$C_4$-Alkoxy oder eine sekundäre Aminogruppe stehen,
mit einem Mol eines Diamins der Formel

$H_2N$-A-$NH_2$ (III),

worin A die oben angegebene Bedeutung hat,
dadurch gekennzeichnet, dass die Umsetzung in Gegenwart von 1,2,3-Trimethylbenzol als Lösungsmittel durchgeführt wird.

2. Verfahren gemäss Anspruch 1, dadurch gekennzeichnet, dass in den Formeln I und II X, $X_1$ und $X_2$ Chlor bedeuten.

3. Verfahren gemäss Anspruch 1, dadurch gekennzeichnet, dass in der Formel II V eine Gruppe der Formel

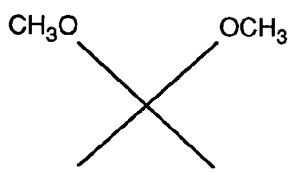

bedeutet.

4. Verfahren gemäss Anspruch 1, dadurch gekennzeichnet, dass in den Formeln I und III A eine Gruppe der Formel

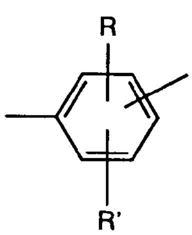 oder 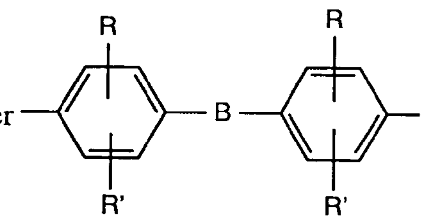

ist, worin die R und R′ die in Anspruch 1 angegebene Bedeutung haben und B -N=N-, -CONH-, -NHCONH- oder -CONHNHCO- bedeutet.

5. Verfahren gemäss Anspruch 4, dadurch gekennzeichnet, dass in den Formeln I und III A eine Gruppe

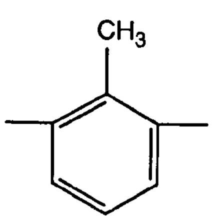 oder 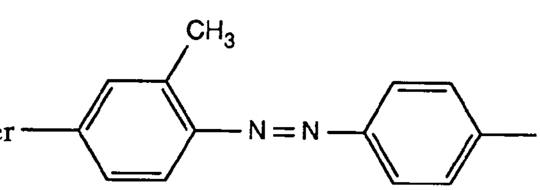

bedeutet.

6. Verfahren gemäss Anspruch 1, dadurch gekennzeichnet, dass die Umsetzung bei einer Temperatur zwischen 40 und 100°C durchgeführt wird und die erhaltenen Pigmente anschliessend bei einer Temperatur zwischen 140 und 160°C konditioniert werden.

Europäisches Patentamt

# EUROPÄISCHER RECHERCHENBERICHT

Nummer der Anmeldung

EP 91810755.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| A | DE - B - 1 569 773 (J.R. GEIGY AG) * Patentansprüche; Spalte 2, Zeilen 11-60 * -- | 1,2,6 | C 09 B 57/04<br>C 07 D 209/50<br>C 09 B 56/00<br>C 09 B 23/10 |
| A | CH - A - 644 391 (DAINIPPON INK AND CHEMICALS INC.) * Patentanspruch 1; Tabelle 2 * -- | 1 | |
| D,A | US - A - 4 006 162 (MODEL et al.) * Spalte 1, Zeile 1 - Spalte 2, Zeile 20; Spalte 4, Zeilen 15-64 * ---- | 1,2,6 | |

RECHERCHIERTE SACHGEBIETE (Int. Cl.⁵)

C 09 B
C 07 D 209/00

Der vorliegende Recherchenbericht wurde für alle Patentansprüche erstellt.

| Recherchenort | Abschlußdatum der Recherche | Prüfer |
|---|---|---|
| WIEN | 17-12-1991 | HAUSWIRTH |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82